(19) Europäisches Patentamt
European Patent Office
Office européen des brevets

(11) Veröffentlichungsnummer: **0 193 103**
**B1**

(12) # EUROPÄISCHE PATENTSCHRIFT

(45) Veröffentlichungstag der Patentschrift: **08.11.89**

(51) Int. Cl.[4]: **C07C 127/00**, C09D 5/44

(21) Anmeldenummer: **86102166.5**

(22) Anmeldetag: **19.02.86**

(54) **Verfahren zur Herstellung einer Vernetzungskomponente für kationische Lackbindemittel.**

(30) Priorität: **27.02.85 AT 572/85**
**22.07.85 AT 2158/85**

(43) Veröffentlichungstag der Anmeldung:
**03.09.86 Patentblatt 86/36**

(45) Bekanntmachung des Hinweises auf die Patenterteilung:
**08.11.89 Patentblatt 89/45**

(84) Benannte Vertragsstaaten:
**DE FR GB IT SE**

(56) Entgegenhaltungen:
**DE-A- 2 313 554**

(73) Patentinhaber: **VIANOVA KUNSTHARZ AKTIENGESELLSCHAFT, Postfach 191 Leechgasse 21, A-8011 Graz(AT)**

(72) Erfinder: **Paar, Willibald, Dr., Haberlandtweg 23, A-8045 Graz(AT)**
Erfinder: **Aigner, Hansjörg, Dr., Keplerstrasse 36a, A-8020 Graz(AT)**

**EP 0 193 103 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung einer Härterkomponente zur Vernetzung von kationischen Lackbindemitteln, wobei diese Härterkomponente ein substituiertes Harnstoffderivat ist.

Kationische Lackbindemittel auf verschiedener Basis werden in ihrer protonisierten, wasserverdünnbaren Form insbesonders im Elektrotauchlackierverfahren (K-ETL-Verfahren) eingesetzt und ergeben dabei entweder korrosionsfeste Grundierungen oder derzeit noch im geringeren Ausmaß dekorative Einschichtlackierungen.

Bekannterweise stellt die für andere Lacksysteme übliche Härtung durch Aminoharze oder Phenolharze aufgrund des basischen Charakters des applizierten Films keine optimale Lösung dar.

In der US-PS 29 95 531 wird daher vorgeschlagen, kationische Systeme durch verkappte Polyisocyanate, welche bei der Einbrenntemperatur entkappen, zur Vernetzung heranzuziehen. Diese Methode wird gemäß DE-20 57 799 auch für die durch das K-ETL-Verfahren applizierten Lackfilme eingesetzt.

Eine andere Art der Härtung von kationischen Lackbindemitteln wird in der EP-B1-00 12 463 beschrieben, wobei in diesen Fällen Umesterungen oder gemäß anderen Literaturstellen Umamidierungen mit aktivierten Estergruppierungen als Vernetzungsmechanismus dienen.

Für die genannten Arten von Vernetzungsreaktionen ist eine relativ hohe Vernetzungstemperatur von über 170°C notwendig sowie der Nachteil von relativ hohen Abspaltverlusten, welche bis zu 20 % des Filmgewichtes betragen können, gegeben.

Es wurde nun gefunden, daß man Harnstoffderivate herstellen kann, welche als Vernetzungskomponente für kationische Lackbindemittel geeignet sind und die Nachteile der Produkte des Standes der Technik in wesentlich verringertem Ausmaß aufweisen.

Die vorliegende Erfindung betrifft demgemäß ein Verfahren zur Herstellung einer Vernetzungskomponente für Hydroxyl- und/oder primäre oder sekundäre Aminogruppen aufweisende kationische Lackbindemittel, welches dadurch gekennzeichnet ist, daß man vorzugsweise in aprotischen Lösemitteln

(A1) β-Hydroxyamine der Formel

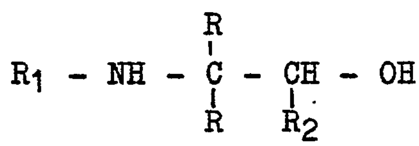

(I)

wobei R ein Wasserstoffatom, ein Alkyl- oder ein Hydroxymethylrest,
$R_1$ ein Alkyl- ein Hydroxyalkyl- Hydroxyalkylester, ein Hydroxypolyoxylalkylrest oder ein Rest der Formel

$-CH_2 - CH(R_4) - CO - R_3-$,

$R_2$ ein Wasserstoffatom, ein Alkylrest oder ein nach Reaktion einer Monoepoxidverbindung

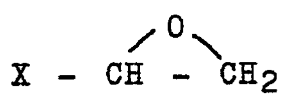

mit einer primären Aminogruppe verbleibender Rest X,
$R_3$ ein - O - Alkylrest, - O - Hydroxylalkylenrest, - O - Hydroxypolyoxyalkylenrest oder $-NH_2$ und
$R_4$ ein Wasserstoffatom oder eine Methylgruppe ist, oder
(A2) β-Hydroxyamine der Formeln

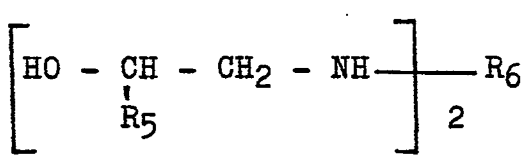

(II)

oder

$$\left[ R_7 - NH - CH_2 - \underset{}{\overset{OH}{\overset{|}{CH}}} - CH_2 - O \right]_2 R_8 \qquad (III)$$

oder

$$\left[ HO - \underset{R_7}{\underset{|}{CH}} - CH_2 - NH - CH_2 - \underset{R_4}{\underset{|}{CH}} - CO - O \right]_{2\ \text{bis}\ 4} R_9 \qquad (IV)$$

wobei $R_5$ jeweils einen Rest $R_2$ oder einen Rest $R_1$ und einen Monoepoxidrest $R_2$,
$R_6$ einen Alkylen- oder einen Polyoxyalkylenrest
$R_7$ einen Alkylrest oder einen Hydroxyalkylrest
$R_8$ einen aliphatischen und/oder aromatischen Rest eines Di- oder Polyglycidylethers und
$R_9$ den Rest eines Polyols mit 2 bis 4 Hydroxylgruppen darstellt,
mit

(B) Isocyanatverbindungen bei 30 bis 60°C in einem Verhältnis umsetzt, daß pro sekundärer Aminogruppe der Formel (I) eine Isocyanatgruppe eingesetzt wird, mit der Maßgabe, daß für β-Hydroxyamine der Gruppe (A1) im wesentlichen Di- oder Polyisocyanatverbindungen und für β-Hydroxyamine der Gruppe (A2) im wesentlichen Monoisocyanatverbindungen eingesetzt werden und

(C) das Reaktionsprodukt bei 80 bis 130°C in Gegenwart eines basischen Katalysators mit 0,5 bis 1 Mol Formaldehyd pro eingesetzter Isocyanatgruppe und der den verbleibenden β-Hydroxyaminen äquimolaren Menge Formaldehyd unter azeotroper Entfernung einer der eingesetzten Formaldehydmenge äquivalenten Menge an Reaktionswasser reagiert.

Die erfindungsgemäß hergestellten Vernetzungskomponenten ergeben in Kombination mit Hydroxyl- und/oder primäre oder sekundäre Aminogruppen tragenden kationischen Harzkomponenten, vorzugsweise solchen auf Basis von Epoxid-Amin-Addukten oder ähnliche Molekülbausteine aufweisenden Produkten Bindemittel, welche bereits bei Temperaturen ab 150°C eingebrannt werden können. Durch die Struktur der Vernetzungskomponente wird eine Verbesserung der Haftungseigenschaften der eingebrannten Lackfilme erzielt.

Die Erfindung betrifft weiters die Verwendung der nach diesem Verfahren hergestellten Vernetzungskomponenten im Kombination mit Hydroxylgruppen und/oder primäre oder sekundäre Aminogruppen aufweisenden und nach Protonierung wasserverdünnbaren Basisharzen als Bindemittel für wasserverdünnbare Lacke, insbesonders für kathodisch abscheidbare Elektrotauchlacke.

Als β-Hydroxyamine der Gruppe (A1) kommen sekundäre Amine, wie Diethanolamin, Diisopropanolamin und homologe β-Hydroxyamine zur Verwendung. Vorzugsweise werden β-Hydroxyamine mit sekundären Hydroxylgruppen eingesetzt. Vorteilhaft können anstelle dieser einfachen Amine Umsetzungsprodukte von primären Alkylaminen mit Monoepoxidverbindungen, wie Glycidylester von Monocarbonsäuren, eingesetzt werden. Eine andere brauchbare Gruppe sind die MICHAEL-Addukte von Acrylsäureestern oder Acrylamid an primäre β-Hydroxymonoamine, wie Monoethanolamin oder Monoisopropanolamin und weitere homologe β-Hydroxyamine.

Amine der Gruppe (A2), d. h. Amine mit zwei oder mehr β-Hydroxyaminogruppierungen werden beispielsweise erhalten

(a) durch Umsetzung von N-Ethanol-alkylendiaminen wie N-Ethanol-ethylendiamin (Aminoethyl-ethanolamin) oder entsprechenden Alkylenhomologen oder primären Alkylendiaminen, wie Ethylendiamin und dessen Homologen, insbesonders Hexamethylendiamin mit Monoepoxidverbindungen, insbesonders Monocarbonsäureglycidylestern;

(b) durch Umsetzung von primären Alkylmonoaminen mit 4 oder mehr C-Atomen mit Di- oder Polyglycidylverbindungen, wie Diglycidylether von aliphatischen oder aromatischen Di- oder Polyhydroxyverbindungen, beispielsweise Glykolen, Diphenolen oder Phenolnovolaken, vorzugsweise von Polyalkylenglykolen;

(c) durch Umsetzung von primären β-Hydroxyaminen mit Di- oder Polyacrylaten wie Di-Propylenglykoldiacrylat oder Trimethylolpropantriacrylat.

Als Isocyanatverbindungen werden beim Einsatz von Aminen der Gruppe (A1), d. h. β-Hydroxyaminen, welche nur eine β-Hydroxygruppierung aufweisen, zur Erzielung der für eine Vernetzung ausreichenden Funkionalität Di- oder Polyisocyanate eingesetzt. Beispielsweise seien hier die aromatischen Diisocyanate, wie Toluylendiisocyanat, aliphatische Diisocyanate wie das Hexamethylendiisocyanat oder

cycloaliphatische Diisocyanate, wie das Isophorondiisocyanat genannt. Als höherfunktionelle Isocyanatverbindungen kommen hier die bekannten Addukte von Diisocyanaten an Polyole zur Verwendung.

Als Monoisocyanate beim Einsatz von Aminen der Gruppe (A2) können Monoisocyanate, wie Phenylisocyanat oder Alkylisocyanate, sowie halbblockierte Diiosocyanate, z. B. aus äquimolaren Mengen Toluylendiisocyanat und 2-Ethylhexanol oder Hydroxyoxazolidinen verwendet werden.

Gegebenenfalls können durch den Einsatz von Kombinationen von Diisocyanaten und Aminen der Gruppe (A2) auch höhermolekulare Strukturen wie

$$(A1)-\left[ DI - (A2) \right]_n - DI - (A1)$$

oder

(A2 – DI – (A2)

aufgebaut werden, wobei DI den Rest eines Diisocyanats und n $\geq$ 1, vorzugsweise 1 ist. Verbleibende β-Hydroxyaminogruppen reagieren bei der Umsetzung mit Formaldehyd zu Oxazolidingruppen.

Die Herstellung der erfindungsgemäßen Vernetzungskomponenten erfolgt durch Umsetzung des β-Hydroxyamins, vorzugsweise in Gegenwart eines aprotischen Lösungsmittels wie Toluol, Xylol oder Methylisobutylketon, mit der Isocyanatverbindung bei 25 bis 40°C. Dabei wird die Isocyanatverbindung dem vorgelegten Amin unter Kühlung langsam zugesetzt. Die Reaktion ist mit dem Ende der Zugabe im wesentlichen beendet und wird durch Bestimmung des NCO-Wertes kontrolliert.

In der zweiten Reaktionsstufe wird das erhaltene Harnstoffderivat mit dem Formaldehyd und nach Zugabe eines basischen Katalysators, z.B. Triethylamin, bei 80 bis 130°C reagiert, wobei das Reaktionswasser durch azeotrope Destillation aus der Reaktionsmasse entfernt wird. Der Formaldehyd wird vorzugsweise in Form des Paraformaldehyds (90 bis 100 % $CH_2O$) eingesetzt. Die Reaktion ist beendet, wenn pro Mol des eingesetzten Formaldehyds 1 Mol Reaktionswasser abgetrennt ist. Das Schleppmittel kann gegebenenfalls nach Ende der Reaktion im Vakuum ganz oder teilweise entfernt werden. Das Produkt wird in der anfallenden Form oder gelöst in geeigneten Lösemitteln, wie Propylenglykolmonomethylether, Diethylenglykoldimethylether oder Methylisobutylketon eingesetzt.

Die Mengenverhältnisse bei der Herstellung der Vernetzungskomponente werden so gewählt, daß in der ersten Stufe pro sekundärer Aminogruppe eine Isocyanatgruppe zur Reaktion gelangt. In der zweiten Stufe wird pro Isocyanatäquivalent 0,5 bis 1 Mol Formaldehyd eingesetzt.

Soferne die Vernetzungskomponenten aufgrund ihres Aufbaues endständige Hydroxylgruppen aufweisen, können die Eigenschaften der solche Vernetzungskomponenten enthaltenden Lacke weiter verbessert werden, wenn man diese Hydroxylgruppen mit halbblockierten Diisocyanaten aufweisenden Monoisocyanaten umsetzt. Die Verbesserungen betreffen insbesondere die Härtungsbedingungen und die Haftung der Folgeschichten.

Für diese besondere Ausführungsform der vorliegenden Erfindung müssen die in der Komponente (A) eingesetzten β-Hydroxyamine mindestens 2 OH-Gruppen aufweisen. Dazu gehören Diethanolamin, Diisopropanolamin und die homologen β-Hydroxyamine. Vorteilhaft können anstelle dieser einfachen Amine Umsetzungsprodukte von primären Monoalkanolaminen, wie Monoethanolamin und seine Homologen, mit Monoepoxidverbindungen, wie Glycidylester von Monocarbonsäuren, eingesetzt werden. Eine andere brauchbare Gruppe sind MICHAEL-Addukte von Aminoalkylalkandiolen, wie 2-Aminoethylpropandiol-1,3 oder Tris-(hydroxymethyl)-aminomethan und Acrylsäureestern oder Acrylamid.

Die halbblockierten Diisocyanate werden in bekannter Weise durch Umsetzung von Diisocyanaten - vorzugsweise solchen, deren Isocyanatgruppen unterschiedliche Reaktivität aufweisen - mit den für solche Systeme geeigneten Blockierungsmitteln, vorzugsweise aliphatischen Monoalkoholen mit 4 oder mehr C-Atomen, hergestellt.

Die ebenfalls einsetzbaren α,β-ethylenisch ungesättigten Monoisocyanatverbindungen sind entweder als Handelsprodukte verfügbar, wie das Isocyanatoacrylat oder können durch partielle Umsetzung der Diisocyanate mit Hydroxy(meth)acrylaten hergestellt werden.

Die Umsetzung der Monoisocyanatprodukte mit den Hydroxylgruppen tragenden Vernetzungskomponenten erfolgt in Gegenwart von isocyanatinerten Lösemitteln bei 50 bis 70°C bis zu einem NCO-Wert von praktisch 0.

Die Herstellung der Lacke unter Verwendung der erfindungsgemäß hergestellten Vernetzungskomponente erfolgt in bekannter Weise durch Mischen mit der Basisharz komponente bei mäßig erhöhter Temperatur. Die erfindungsgemäß hergestellten Vernetzungskomponenten werden dabei in einer Menge von 10 bis 40 Gew.-%, vorzugsweise 15 bis 30 Gew.-%, bezogen auf das Gesamtbindemittel eingesetzt. Die Formulierung der Lacke, ihre Pigmentierung, Herstellung und Verarbeitung, insbesonders nach dem kathodischen Elektrotauchverfahren ist dem Fachmann bekannt und bedarf keiner weiteren Erläuterung.

Die folgenden Beispiele erläutern die Erfindung, ohne sie in ihrem Umfang zu beschränken. Alle Angaben in Teilen oder Prozenten beziehen sich, soferne nichts anderes angegeben ist, auf Gewichtseinheiten.

Als β-Hydroxyamine werden in den Beispielen folgende Verbindungen eingesetzt.

Formel I:

AM 1 Diisopropanolamin (Molgew. 133)
AM 2 Umsetzungsprodukt aus äquimolaren Mengen 2-Ethylhexylamin und CE (Molgew. 379)
(CE = Glycidylester von verzweigten $C_9$-$C_{11}$- Monocarbonsäuren)
AM 3 Umsetzungsprodukt aus äquimolaren Mengen Monoethanolamin und CE (Molgew. 311)
AM 4 Umsetzungsprodukt aus äquimolaren Mengen Monoisopropanolamin und 2-Ethylhexylacrylat (Molgew. 259)
AM 5 Umsetzungsprodukt aus 1 Mol Aminoethylpropandiol und 1 Mol Octenoxid (Molgew. 247)
AM 6 Umsetzungsprodukt aus 1 Mol Tris(hydroxymethyl)aminomethan und 1 Mol n-Butylacrylat (Molgew. 249)

Formel II:

AM 7 Umsetzungsprodukt aus äquimolaren Mengen Aminoethylethanolamin und Dodecenoxid (Molgew. 288)
AM 8 Umsetzungsprodukt aus 1 Mol 1,6-Hexamethylendiamin und 2 Mol CE (Molgew. 616)
AM 9 Umsetzungsprodukt aus 1 Mol Trimethylhexamethylendiamin und 2 Mol Phenylglycidylether (Molgew. 458)

Formel III:

AM 10 Umsetzungsprodukt aus 1 Mol eines Polypropylenglykoldiglycidylethers (Expoxidäquivalentgew. ca. 200) mit 2 Mol 2-Ethylhexylamin (Molgew. 658)
AM 11 Umsetzungsprodukt aus 1 Mol eines Polypropylenglykoldiglycidylethers (Epoxidäquivalentgewicht ca. 200) mit 2 Mol Monoisopropanolamin (Molgew. 550).
AM 12 Umsetzungsprodukt aus 1 Mol eines Epoxidharzes auf Basis von Bisphenol A (Epoxidäquivalentgewicht ca. 190) mit 2 Mol Isobutylamin (Molgew. 546)

Formel IV

AM 13 Umsetzungsprodukt aus 2 Mol Monoisopropanolamin und 1 Mol Hexandioldiacrylat (Molgew. 378)
AM 14 Umsetzungsprodukt aus 1 Mol Trimethylolpropantriacrylat und 3 Mol Monoethanolamin (Molgew. 479)
AM 15 Umsetzungsprodukt aus 1 Mol Butandioldiacrylat und 2 Mol Monoisopropanolamin (Molgew. 348)

Als Isocyanatverbindungen werden in den Beispielen eingesetzt:

IC 1 Toluylendiisocyanat (handelsübliches Isomerengemisch)
IC 2 Isophorondiisocyanat
IC 3 Trimethylhexamethylendiisocyanat
IC 4 Phenylisocyanat
IC 5 mit 2-Ethylhexanol halbverkapptes Toluylendiisocyanat

Als halbblockierte Diisocyanate werden folgende Produkte verwendet

MIC 1 IC 1 / 2-Ethylhexanol (Molgew. 304)
MIC 2 IC 2 / Hydroxyethylacrylat (Molgew. 338)
MIC 3 IC 1 / Hydroxyethylmethacrylat (Molgew. 304)
MIC 4 IC 2 / Acetessigester (Molgew. 352)
MIC 5 IC 2/ Hydroxyethyloxazolidin (Molgew. 339) (70%ige Lösung in Diethylenglykoldimethylether)

<u>Beispiele 1 bis 6:</u> In einem geeigneten Reaktionsgefäß wird das β-Hydroxyamin (AM), verdünnt mit etwa 50 % seines Gewichtes Toluol (1), vorgelegt und bei 25 bis maximal 40°C unter ausreichender Kühlung portionsweise mit der Isocyanatverbindung (IC) versetzt. Die Reaktion ist mit Ende der Zugabe üblicherweise abgeschlossen (NCO-Wert praktisch 0). Nach Aufwärmen auf ca. 70°C wird der Paraformaldehyd und 0,01 - 0,1 Gew.-% (bezogen auf den Feststoffanteil) Triethylamin zugegeben und die Temperatur erhöht bis sich durch das vorgelegte Toluol eine gleichmäßige Rückflußdestillation unter Abtrennung des

Reaktionswassers eingestellt hat. Die Destillation wird solange fortgesetzt, bis die berechnete Wassermenge (1 Mol Wasser pro 1 Mol eingesetztem Formaldehyd, 100%) gebildet ist. Anschließend wird das Schleppmittel unter Vakuum abdestilliert und der Ansatz im angegebenen Lösemittel gelöst. Als Lösemittel werden in den Beispielen Propylenglykolmonomethylether (PM), Diethylenglykoldimethylether (DGDE), Methylisobutylketon (MIBK) und Xylol (X) eingesetzt.

Mengen und Art der Ausgangsstoffe sind in der Tabelle 1 zusammengefaßt.

(1) Anstelle von Toluol kann in gleicher Weise auch Methylisobutylketon eingesetzt werden.

Tabelle 1

| Beispiel | AM Gew.-Teile (Mol) | IC Gew.-Teile (Mol) | Formaldehyd Gew.-Teile (Mol) | OH/MOL | MIC Gew.-Teile (Mol) | Lösemittel %Festkörper | Molgew. (berechn.) |
|---|---|---|---|---|---|---|---|
| 1 | 266 AM 1 (2,0)<br>616 AM 8 (1,0) | 348 IC 1 (2,0) | 120 (4,0) | 4 | 1064 MIC 1 (3,5) | 75 DGDE | 2342 |
| 2 | 494 AM 5 (2,0) | 210 IC 3 (1,0) | 60 (2,0) | 4 | 1014 MIC 2 (3,0) | 80 MIBK | 1742 |
| 3 | 758 AM 2 (2,0)<br>616 AM 8 (1,0) | 444 IC 2 (2,0) | 120 (4,0) | – | – | 85 PM | 1866 |
| 4 | 266 AM 1 (2,0)<br>616 AM 8 (1,0) | 348 IC 1 (2,0) | 120 (4,0) | 2 | – | 80 PM | 1278 |
| 5 | 550 AM 11 (1,0)<br>494 AM 5 (2,0) | 444 IC 1 (2,0) | 120 (4,0) | 6 | 1216 MIC 1 (4,0) | 70 X | 2752 |
| 6 | 518 AM 4 (2,0) | 210 IC 3 (1,0) | 60 (2,0) | – | – | 85 DGDE | 752 |
| 7 | 622 AM 3 (2,0) | 210 IC 3 (1,0) | 60 (2,0) | 2 | 634 MIC 4 (1,8) | 75 X | 1490 |
| 8 | 378 AM 13 (1,0) | 608 IC 5 (2,0) | 60 (2,0) | – | – | 90 PM | 1010 |
| 9 | 518 AM 4 (2,0)<br>288 AM 7 (1,0) | 420 IC 3 (2,0) | 120 (4,0) | 2 | – | 80 PM | 1274 |
| 10 | 498 AM 6 (2,0) | 174 IC 1 (1,0) | 60 (2,0) | 4 | 1064 MIC 3 (3,5) | 75 DGDE | 1760 |
| 11 | 658 AM 10 (1,0) | 608 IC 5 (2,0) | 60 (2,0) | – | – | 80 PM | 1290 |
| 12 | 550 AM 11 (1,0)<br>622 AM 3 (2,0) | 420 IC 3 (2,0) | 120 (4,0) | 4 | 1082 MIC 2 (3,2) | 70 MIBK | 2722 |
| 13 | 546 AM 12 (1,0)<br>622 AM 3 (2,0) | 420 IC 3 (2,0) | 120 (4,0) | 2 | 542 MIC 5 (1,6) | 65 DGDE | 2178 |
| 14 | 479 AM 14 (1,0) | 357 IC 4 (3,0) | 90 (3,0) | – | – | 70 MIBK | 872 |
| 15 | 348 AM 15 (1,0) | 608 IC 5 (2,0) | 60 (2,0) | – | – | 70 DGDE | 980 |

Für die Prüfung der Vernetzungskomponenten werden Mischungen mit kationischen Bindemitteln hergestellt. Als solche, nach Protonierung wasserlösliche Basisharze werden modifizierte Epoxidharz-Amin-Addukte, kationisch modifizierte Epoxidharz-Acrylsäureester (B 4), resolhärtbare Bindemittel auf Basis eines modifizierten Polybutadienöles (B 5) und oxazolidinmodifizierte Epoxidharze (B 6) eingesetzt. Das Produkt (B 6) wird auch in Kombination mit den anderen Basisharzen als Anreibeharz bei der Lackherstellung eingesetzt. Die Herstellung der Basisharze erfolgt in der nachstehend beschriebenen Weise:

(B 1): In einem mit Rührer, Thermometer und Rückflußkühler ausgestattetem Reaktionsgefäß werden 1000 g eines Epoxidharzes (Basis Bisphenol-A; Epoxyäquivalentgewicht ca. 500) in 512 g Ethylenglykolmonoethylether bei 60 bis 70°C gelöst. Anschließend werden 37 g Diethylamin und 158 g Diethanolamin zugegeben und der Ansatz 3 Stunden bei 100°C reagiert. Das Produkt hat eine OH-Zahl entsprechend 375 mg KOH/g.

(B 2): Zu einer Lösung von 1000 g eines Epoxidharzes (Basis Bisphenol A; EG ca. 500) in 551 g Methylisobutylketon werden bei 70°C 168 g Isononansäure, 53 g Diethanolamin und 33 g Diethylaminopropylamin zugegeben und das Reaktionsgemisch bei 95 bis 100°C so lange gehalten, bis eine Säurezahl von unter 3 mg KOH/g erreicht ist (Hydroxylzahl = 270 mg KOH/g).

(B 3): 400 g eines Epoxidharzes (Bisphenol A; EG 200) werden in 261 g Ethylenglykolmonomethylether gelöst und mit 278 g eines Halbesters aus Phthalsäureanhydrid und 2-Ethylhexanol, 67 g Diisopropanolamin und 37 g Diethylamin bei 90 bis 95°C umgesetzt, bis eine Säurezahl von weniger als 3 mg KOH/g erreicht ist (Hydroxylzahl = 230 KOH/g).

(B 4): In einem mit Rührer, Thermometer und Rückflußkühler ausgestatteten Reaktionsgefäß werden 1000 g eines Epoxidharzes auf Bisphenol A-Basis (Epoxidäquivalent ca. 500) in 492 g Ethylglykolacetat bei 60 bis 70°C gelöst, 0,2 g Hydrochinon und 144 g Acrylsäure zugegeben und die Temperatur auf 100 bis 110°C gesteigert. Die Reaktion wird bei dieser Temperatur bis zu einer Säurezahl von unter 5 mg KOH/g geführt (DBZ = 1,75). Anschließend wird das Reaktionsprodukt bei 60 bis 70°C mit 652 g BMI (70 %) *) versetzt und bis zu einem NCO-Wert von praktisch Null reagiert (DBZ = 1,25, BNZ = 1,1).

*) Basisches Monoisocyanat aus 1 Mol Toluylendiisocyanat und 1 Mol Diethylethanolamin (70%ig in Methylisobutylketon)
DBZ: Doppelbindungszahl: entspricht der Anzahl der endständigen ethylenischen Doppelbindungen pro 1000 Molekulargewichtseinheiten
BNZ: Basenzahl: entspricht der Anzahl der basischen N-Gruppierungen pro 1000 Molekulargewichtseinheiten

(B 5): 700 Tle B 180 **) werden in bekannter Weise in Gegenwart von 0,5 Tlen Diphenylparaphenylendiamin (Inhibitor) bei 200°C mit 100 Tlen Maleinsäureanhydrid so lange reagiert, bis dieses vollständig gebunden ist. Nach Kühlen auf 100°C werden 130 Tle 2-Ethylhexanol zugesetzt und bei 120°C bis zum Erreichen der theoretischen Säurezahl des Halbesters verestert (MAD A).
110 Tle MAD A (entsprechend ca. 0,12 COOH-Gruppen) werden mit 212 Tlen eines Bisphenol A-Diepoxidharzes (Epoxidäquivalent ca. 190) in 80%iger Lösung in Diethylenglykoldimethylether bei 120°C bis zu einer Säurezahl von praktisch Null umgesetzt. Nach Zusatz von 108 Tlen Diethylenglykoldimethylether, 59 Tlen Diethylaminopropylamin (0,45 Mol) und 59 Tlen 2-Ethylhexylamin (0,45 Mol) wird der Ansatz bei 65 bis 70°C bis zu einem Epoxidwert von praktisch Null reagiert. Nach Erreichen dieses Wertes werden 114 Tle Bisphenol A (0,5 Mol) und 50 Tle Paraformaldehyd, 91 % (1,5 Mol) zugegeben und die Reaktion bei 60°C bis zum Erreichen eines Gehaltes an freiem Formaldehyd von 0,5 bis 1 % geführt.

**) B 180 ist ein flüssiges Polybutadienöl (ca. 75 % 1,4 cis-, ca. 24 % 1,4-trans- und ca. 1 % Vinyl-Doppelbindungen; Molekulargewicht ca. 1500 ± 15 % Jodzahl ca. 450 g/ 1000 g).

(B 6): 500 Tle eines Epoxidharzes auf Basis von Bisphenol A (Epoxidäquivalentgewicht ca. 500) werden in 214 Tlen Propylenglykolmonomethylether gelöst und bei 110°C mit 83 Tlen eines Halbesters aus Phthalsäureanhydrid und 2-Ethylhexanol in Gegenwart von 0.5 g Triethylamin als Katalysator bis zu einer Säurezahl von weniger als 3 mg KOH/g reagiert. Dann werden 120 Tle eines NH-funktionellen Oxazolidins aus Aminoethylethanolamin, 2-Ethylhexylacrylat und Formaldehyd, sowie 26 Tle Diethylaminopropylamin zugefügt und der Ansatz bei 80°C bis zu einem Epoxidwert von praktisch 0 reagiert. Der Ansatz wird mit 200 Tlen Propylenglykolmonomethylether verdünnt und mit 97 Tlen 3 n-Ameisensäure partiell neutralisiert.

Aus den in Tab. 2 angegebenen Bindemittelmischungen wurden Lacke entsprechend den in dieser Tabelle gemachten Angaben hergestellt und nach Neutralisation mit Ameisensäure und Verdünnen mit deionisiertem Wasser auf einen Festkörpergehalt von 18 % auf zinkphosphatiertem Stahlblech in bekannter Weise elektrisch abgeschieden. Bei der angegebenen Einbrenntemperatur zeigen beschichtete Bleche mit einer Filmstärke von 23 ± 2 µm eine Salzsprühbeständigkeit nach ASTM -B 117-64 von mehr als 1000 Stunden (Ablösung am Kreuzschnitt unter 2 mm).

Die eingesetzte Farbpaste setzt sich aus 100 Tlen Anreibeharz, (fest) 1 Tl Farbruß, 12 Tlen basisches Bleisilikat und 147 Tlen Titandioxid zusammen. Die Herstellung des Anreibeharzes erfolgt in folgender Weise: 500 Tle eines Epoxidharzes aus Basis von Bisphenol A (Epoxidäquivalentgewicht ca. 500) werden in 214 Tlen Propylenglykolmonomethylether gelöst und bei 110°C mit 83 Tlen eines Halbesters aus Phthalsäureanhydrid und 2-Ethylhexanol in Gegenwart von 0,5 g Triethylamin als Katalysator bis zu einer Säurezahl von weniger als 3 mg KOH/g reagiert. Dann werden 120 Tle eines NH-funktionellen Oxazolidins aus Aminoethylethanolamin, 2-Ethylhexylacrylat und Formaldehyd, sowie 26 Tle Diethylaminopropylamin zugefügt und der Ansatz bei 80°C bis zu einem Epoxidwert von praktisch 0 reagiert. Der Ansatz wird mit 200 Tlen Propylenglykolmonomethylether verdünnt und mit 97 Tlen 3-normaler Ameisensäure partiell neutralisiert. Der resultierende Festkörpergehalt beträgt 58,8.%.

Tabelle 2

| Bindemittelmischung | | Lackzusammensetzung | | | | Einbrennbedingung |
|---|---|---|---|---|---|---|
| Basisharz Tle | Vernetzungskomponente Tle aus Beispiel | Bindemittelmischung (1) | Pigmentpaste entspr. 25 Tlen Festharz + 40 Tlen Pigment | Neutralisationsmittel (2) gesamt | Katalysator (3) | 20 Min/°C |
| 70 B 6 | 30 / 1 | 119 | 83 | 35 | 0,5 Sn | 160 |
| 80 B 5 | 20 / 10 | 100 | 83 | 40 | 0,5 Pb | 160 |
| 85 B 2 | 15 / 7 | 107 | 83 | 45 | – | 150 |
| 75 B 1 | 25 / 4 | 103 | 83 | 45 | 0,4 Co | 160 |
| 70 B 2 | 30 / 2 | 103 | 83 | 45 | – | 170 |
| 80 B 2 | 20 / 6 | 103 | 83 | 40 | 0,3 Mn | 170 |
| 75 B 4 | 25 / 2 | 103 | 83 | 45 | 0,1 Co | 150 |
| 85 B 5 | 15 / 12 | 101 | 83 | 45 | – | 160 |
| 70 B 2 | 30 / 11 | 101 | 83 | 35 | 0,5 Co | 160 |
| 75 B 5 | 25 / 5 | 102 | 83 | 40 | – | 160 |
| 85 B 3 | 15 / 3 | 98 | 83 | 45 | 0,5 Co | 180 |
| 75 B 6 | 25 / 12 | 123 | 83 | 30 | 0,3 Pb | 150 |
| 70 B 1 | 30 / 8 | 100 | 83 | 40 | 0,2 Co | 160 |
| 80 B 4 | 20 / 10 | 105 | 83 | 40 | – | 170 |
| 80 B 2 | 20 / 9 | 104 | 83 | 40 | 0,2 Mn | 170 |
| 75 B 5 | 25 / 14 | 105 | 83 | 40 | 0,5 Pb | 170 |
| 80 B 6 | 20 / 13 | 125 | 83 | 35 | 0,4 Sn | 150 |
| 70 B 6 | 30 / 15 | 121 | 83 | 35 | 0,2 Co | 170 |

(1) Entsprechend 75 Tlen Festharz
(2) Millimol Ameisensäure/100 g Festharz
(3) % Metall / Festharz

## Patentansprüche

1. Verfahren zur Herstellung einer Vernetzungskomponente für Hydroxyl- und/oder primäre oder sekundäre Aminogruppen aufweisende kationische Lackbindemittel, dadurch gekennzeichnet, daß man vorzugsweise in aprotischen Lösemitteln

(A1) β-Hydroxyamine der Formel

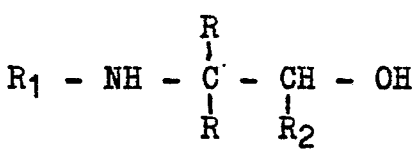

(I)

wobei R ein Wasserstoffatom, ein Alkyl- oder ein Hydroxymethylrest,

$R_1$ ein Alkyl- ein Hydroxyalkyl- Hydroxyalkylester- ein Hydroxypolyoxyalkylrest oder ein Rest der Formel

$-CH_2 - CH(R_4) - CO - R_3$.,

$R_2$ ein Wasserstoffatom, ein Alkylrest oder ein nach Reaktion einer Monoepoxidverbindung

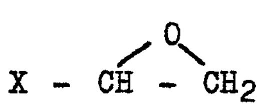

mit einer primären Aminogruppe verbleibender Rest X,

$R_3$ ein - O - Alkylrest, - O - Hydroxyalkylenrest, - O - Hydroxypolyoxyalkylenrest oder $-NH_2$ und

$R_4$ ein Wasserstoffatom oder eine Methylgruppe ist, oder
(A2) β-Hydroxyamine der Formeln

$$\left[HO - \underset{\textstyle R_5}{CH} - CH_2 - NH\right]_2 R_6 \qquad (II)$$

oder

$$\left[R_7 - NH - CH_2 - \overset{\textstyle OH}{CH} - CH_2 - O\right]_2 R_8 \qquad (III)$$

oder

$$\left[HO - \underset{\textstyle R_7}{CH} - CH_2 - NH - CH_2 - \underset{\textstyle R_4}{CH} - CO - O\right]_{2\ bis\ 4} R_9 \qquad (IV)$$

wobei $R_5$ jeweils einen Rest $R_2$ oder einen Rest $R_1$ und einen Monoepoxidrest $R_2$,
$R_6$ einen Alkylen- oder einen Polyoxylalkylenrest
$R_7$ einen Alkylrest oder einen Hydroxyalkylrest
$R_8$ einen aliphatischen und/oder aromatischen Rest eines Di- oder Polyglycidylethers und
$R_9$ den Rest eines Polyols mit 2 bis 4 Hydroxylgruppen darstellt,
mit
(B) Isocyanatverbindungen bei 30 bis 60°C in einem Verhältnis umsetzt, daß pro sekundärer Aminogruppe der Formel (I) eine Isocyanatgruppe eingesetzt wird, mit der Maßgabe, daß für β-Hydroxyamine der Gruppe (A1) im wesentlichen Di- oder Polyisocyanatverbindungen und für β-Hydroxyamine der Gruppe (A2) im wesentlichen Monoisocyanatverbindungen eingesetzt werden und
(C) das Reaktionsprodukt bei 80 bis 130°C in Gegenwart eines basischen Katalysators mit 0,5 bis 1 Mol Formaldehyd pro eingesetzter Isocyanatgruppe und der den verbleibenden β-Hydroxyaminen äquimolaren Menge Formaldehyd unter azeotroper Entfernung einer der eingesetzten Formaldehydmenge äquivalenten Mengen an Reaktionswasser reagiert.

2. Verfahren nach Anspruch 1, dadurch gekennzeichnet, daß man als β-Hydroxyamine der Formel (I) Umsetzungsprodukte von primären Alkylaminen mit Monoepoxidverbindungen einsetzt.

3. Verfahren nach Anspruch 1, dadurch gekennzeichnet, daß man als β-Hydroxyamine der Gruppe (A1) MICHAEL-Addukte von Acrylsäureestern oder Acrylamid an primäre β-Hydroxymonoamine einsetzt.

4. Verfahren nach Anspruch 1, dadurch gekennzeichnet, daß man als β-Hydroxyamine der Gruppe (A2) Umsetzungsprodukte von N-Ethanol-Alkylendiaminen oder primären Alkylendiaminen mit Monoepoxidverbindungen einsetzt.

5. Verfahren nach Anspruch 1, dadurch gekennzeichnet, daß man als β-Hydroxyamine der Gruppe (A2) Umsetzungsprodukte von primären Alkylmonoaminen mit Di- oder Polyglycidylverbindungen einsetzt.

6. Verfahren nach Anspruch 5, dadurch gekennzeichnet, daß man als Glycidylverbindungen Diglycidylether von Polyalkylenglykolen einsetzt.

7. Verfahren nach Anspruch 1, dadurch gekennzeichnet, daß man als β-Hydroxyamine der Gruppe (A2) Umsetzungsprodukte von primären Hydroxyaminen mit Di- oder Polyacrylaten einsetzt.

8. Verfahren nach den Ansprüchen 1 bis 7, dadurch gekennzeichnet, daß man bei den Vernetzungskomponenten, welche gemäß ihrem Aufbau endständige Hydroxylgruppen aufweisen, diese mit halbblockierten Diisocyanaten und/oder α,β-ethylenisch ungesättigte Gruppen aufweisenden Monoisocyanaten umsetzt.

9. Verfahren nach Anspruch 8, dadurch gekennzeichnet, daß die in der Komponente (A) eingesetzten β-Hydroxyamine mindestens 2 Hydroxylgruppen aufweisen.

10. Verfahren nach den Ansprüchen 8 und 9, dadurch gekennzeichnet, daß man als β-Hydroxyamine Umsetzungsprodukte von primären Monoalkanolaminen mit Monoepoxidverbindungen einsetzt.

11. Verfahren nach den Ansprüchen 8 und 9, dadurch gekennzeichnet, daß man als β-Hydroxyamine MICHAEL-Addukte von Aminoalkylalkandiolen mit Acrylsäureestern oder Acrylamid einsetzt.

12. Verfahren nach Anspruch 1, dadurch gekennzeichnet, daß in der Stufe (C) der Formaldehyd in

Form von Paraformaldehyd mit einem $CH_2O$-Gehalt von 90 bis 100 % eingesetzt wird.

13. Verwendung der nach den Ansprüchen 1 bis 12 hergestellten Vernetzungskomponenten mit Hydroxylgruppen und/oder primäre oder sekundäre Aminogruppen tragenden Basisharzen in einem Verhältnis von 60 bis 90, vorzugsweise 70 bis 85 Gew.-% des Basisharzes zu 10 bis 40, vorzugsweise 15 bis 30 Gew.-% der Vernetzungskomponente.

14. Verwendung der nach den Ansprüchen 1 bis 13 hergestellten Harzkombinationen als Bindemittel für wasserverdünnbare Lacke, insbesonders kathodisch abscheidbare Elektrotauchlacke.

## Claims

1. Process for preparing a crosslinking component for cationic paint binders containing hydroxyl and/or primary or secondary amino groups, characterised in that, preferably in aprotic solvents,

(A1) β-hydroxyamines of formula

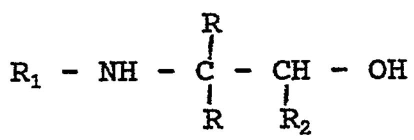

(I)

wherein R represents a hydrogen atom or an alkyl or hydroxymethyl group, $R_1$ represents an alkyl, hydroxyalkyl or hydroxypolyoxyalkyl group or a group of formula $-CH_2-CH(R_4)-CO-R_3$, $R_2$ represents a hydrogen atom, an alkyl group or a group X which remains after the reaction of a monoepoxide compound

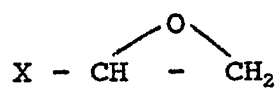

with a primary amino group, $R_3$ represents an –O– alkyl group, –O– hydroxyalkylene group, –O– hydroxypolyoxyalkylene group or $-NH_2$ and $R_4$ is a hydrogen atom or a methyl group, or

(A2) β-hydroxyamines of formula

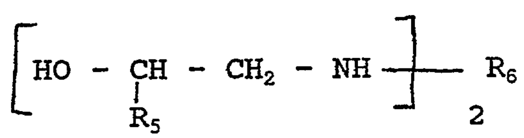

(II)

or

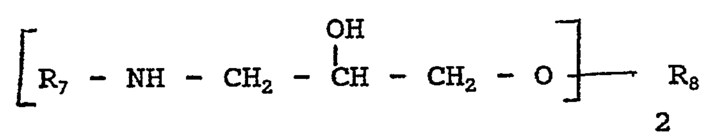

(III)

or

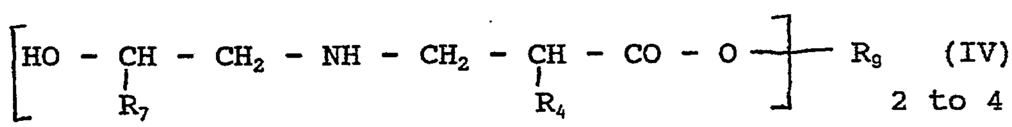

(IV)

wherein $R_5$ in each case represents a group $R_2$ or a group $R_1$ together with a monoepoxide group $R_2$, $R_6$ represents an alkylene or polyoxyalkylene group; $R_7$ represents an alkyl group or a hydroxyalkyl group, $R_8$ represents an aliphatic and/or aromatic group of a di- or polyglycidylether, and $R_9$ represents of a polyol with 2 to 4 hydroxyl groups, are reacted with (B) isocyanate compounds at 30 to 60°C in a ratio such that for each secondary amino group of formula (I) an isocyanate group is used, with the proviso that, for β-hydroxyamines of the group (A1), essentially di- or polysocyanate compounds are used and for β-hydroxyamines of the group (A2) essentially monoisocyanate compounds are used and (C) the reaction product is reacted at 80 to 130°C in the presence of a basic catalyst with 0.5 to 1 mol of formaldehyde for each isocyanate group used and in the presence of a quantity of formaldehyde which is equimolar to the remaining β-hydroxyamines, with azeotropic removal of a quantity of water of reaction equivalent to the quantity of formaldehyde used.

2. Process according to claim 1, characterized in that reaction products of primary alkylamines with monoepoxide compounds are used as the β-hydroxyamines of formula (I).

3. Process according to claim 1, characterized in that MICHAEL adducts of acrylic acid esters or acrylamide with primary β-hydroxymonoamines are used as the β-hydroxyamines of the group (A1).

4. Process according to claim 1, characterized in that reaction products of N-ethanol-alkylenediamines or primary alkylenediamines with monoepoxide compounds are used as the β-hydroxyamines of the group (A2).

5. Process according to claim 1, characterized in that reaction products of primary alkylmonoamines with di- or polyglycidyl compounds are used as the β-hydroxyamines of the group (A2).

6. Process according to claim 5, characterized in that diglycidylethers of polyalkylene glycols are used as the glycidyl compounds.

7. Process according to claim 1, characterized in that reaction products of primary hydroxyamines with di- or polyacrylates are used as the β-hydroxyamines of the group (A2).

8. Process according to claims 1 to 7, characterized in that, in the case of the crosslinking components which have terminal hydroxyl groups according to their structure, the latter are reacted with semi-blocked diisocyanates and/or monoisocyanates containing α,β-ethylenically unsaturated groups.

9. Process according to claim 8, characterized in that the β-hydroxyamines used in component (A) contain at least two hydroxyl groups.

10. Process according to claims 8 and 9, characterized in that reaction products of primary monoalkanolamines with monoepoxide compounds are used as the β-hydroxyamines.

11. Process according to claims 8 and 9, characterized in that MICHAEL adducts of aminoalkylalkanediols with acrylic acid esters or acrylamide are used as β-hydroxyamines.

12. Process according to claim 1, characterized in that, in step (C), the formaldehyde is used in the form of paraformaldehyde with a $CH_2O$ content of 90 to 100%.

13. Use of the crosslinking components prepared according to claims 1 to 12 with basic resins carrying hydroxyl groups and/or primary or secondary amino groups, in a ratio of 60 to 90, preferably 70 to 85, % by weight of the basic resin to 10 to 40, preferably 15 to 30, % by weight of the crosslinking component.

14. Use of the resin combinations prepared according to claims 1 to 13 as binders for water-dilutable paints, particularly cathodically depositable electrodeposition paints.

**Revendications**

1. Procédé pour la préparation d'un constituant de réticulation pour liants cationiques pour vernis présentant des groupes hydroxyle et/ou amine primaires ou secondaires, caractérisé en ce que l'on fait réagir de préférence dans des solvants aprotiques

(A1) des hydroxylamines β de formule

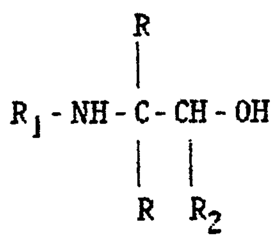

(I)

dans laquelle R représente un atome d'hydrogène, un reste alkyle ou un reste hydroxyméthyle, $R_1$ représente un reste alkyle, hydroxyalkyle, hydroxypolyoxyalkyle ou un reste de formule $-CH_2-CH(R_4)-CO-R_3$, $R_2$ représente un atome d'hydrogène, un reste alkyle ou un reste X demeurant après la réaction d'un composé monoépoxy

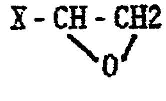

avec un groupe amine primaire, $R_3$ représente un reste –O–alkyle, –O–hydroxyalkylène, –O–hydroxypolyoxyalkylène ou $-NH_2$ et $R_4$ représente un atome d'hydrogène ou un groupe méthyle ou (A2) des hydroxylamines β de formule

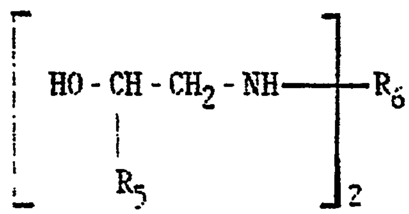

(II)

ou

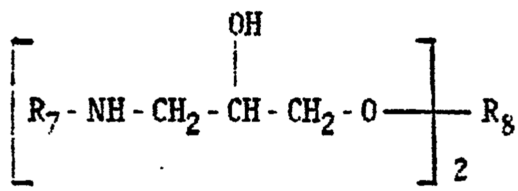

(III)

ou

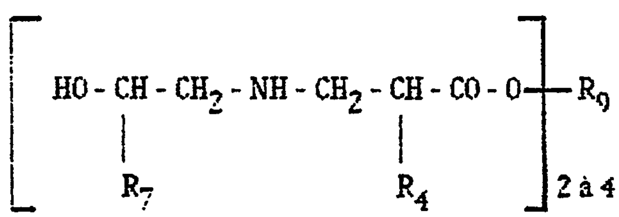

(IV)

ou $R_5$ représente a chaque fois un reste $R_2$ ou un reste $R_1$ et un reste monoépoxyde $R_2$, $R_6$ représente un reste alkylène ou un reste polyoxyalkylène, $R_7$ représente un reste alkyle ou un reste hydroxyalkyle, $R_8$ représente un reste aliphatique et/ou aromatique d'un di- ou polyglycidyléther et $R_9$ représente le reste d'un polyol avec 2 à 4 groupes hydroxyle avec (B) des composés d'isocyanate entre 30 et 60°C dans des proportions telles que, par groupe amine secondaire de formule (I) un groupe isocyanate est utilisé, avec la condition que comme hydroxylamines β du groupe (A1) on utilise essentiellement des composés de di- ou polyisocyanate et que comme hydroxylamines β du groupe (A2) on utilise essentiellement des composés de monoisocyanate et

(C) le produit de la réaction entre 80 et 130°C en présence d'un catalyseur basique avec 0,5 à 1 mole de formaldéhyde par groupe isocyanate introduit et la quantité de formaldéhyde équimolaire des hydroxylamines β restantes avec élimination azéotropique d'une quantité d'eau de réaction équivalente de la quantité de formaldéhyde utilisée.

2. Procédé selon la revendication 1, caractérisé en ce que l'on utilise en tant qu'hydroxylamines β de formule (I) des produits de réaction d'alkylamines primaires avec des composés monoépoxy.

3. Procédé selon la revendication 1, caractérisé en ce que l'on utilise en tant qu'hydroxylamines β du groupe (A1) des produits d'addition de Michael d'esters acryliques ou d'acrylamide et d'hydroxymonoamines β primaires.

4. Procédé selon la revendication 1, caractérisé en ce que l'on utilise en tant qu'hydroxylamines β du groupe (A2) des produits de réaction de N-éthanolalkylènediamines ou d'alkylènediamines primaires avec des composés monoépoxy.

5. Procédé selon la revendication 1, caractérisé en ce que l'on utilise en tant qu'hydroxylamines β du groupe (A2) des produits de réaction d'alkylmonoamines primaires avec des composés di- ou polyglycidyliques.

6. Procédé selon la revendication 5, caractérisé en ce que l'on utilise en tant que composés glycidyliques des diglycidyléthers de polyalkylèneglycols.

7. Procédé selon la revendication 1, caractérisé en ce que l'on utilise en tant qu'hydroxylamines β du groupe (A2) des produits de réaction d'hydroxylamines primaires avec des di- ou polyacrylates.

8. Procédé selon les revendications 1 à 7, caractérisé en ce que s'agissant des constituants de réticulation qui, selon leur structure, présentent des groupes hydroxyle en position terminale, l'on fait réagir ceux-ci avec des diisocyanates semi-bloqués et/ou avec des monoisocyanates présentant des groupes α, β-éthyléniquement insaturés.

9. Procédé selon la revendication 8, caractérisé en ce que les hydroxylamines β utilisés dans le constituant (A) présentent au moins 2 groupes hydroxyle.

10. Procédé selon les revendications 8 et 9, caractérisé en ce que l'on utilise en tant qu'hydroxylamines β des produits de réaction de monoalcanolamines primaires avec des composés monoépoxy.

11. Procédé selon les revendications 8 et 9, caractérisé en ce que l'on utilise en tant qu'hydroxylami-

nes β des produits d'addition de Michael d'aminoalkylalcanédiols avec des esters acryliques ou avec l'acrylamide.

12. Procédé selon la revendication 1, caractérisé en ce que dans l'étape (C), le formaldéhyde est utilisé sous forme de paraformaldéhyde avec une teneur en $CH_2O$ de 90 à 100%.

13. Utilisation des constituants de réticulation préparés selon les revendications 1 à 12 avec des résines de base porteuses de groupes hydroxyle et/ou de groupes amine primaires ou secondaires dans des proportions de 60 à 90, de préférence de 70 à 85% en poids de la résine de base pour 10 à 40, de préférence 15 à 30% en poids du constituant de réticulation.

14. Utilisation des combinaisons de résine préparées selon les revendications 1 à 13 en tant que liants pour vernis diluables à l'eau, en particulier pour vernis de trempage électrophorétique précipitables cathodiquement.